# EUROPEAN PATENT APPLICATION

(11) **EP 2 644 081 A1**
(43) Date of publication of application: **02.10.2013**
(21) Application number: 11843675.7
(22) Date of filing: 14.09.2011
(51) Int. Cl.: A61B 1/00

(54) **SCANNING CONFOCAL ENDOSCOPE SYSTEM**

(30) Priority: 24.11.2010 JP 2010261141
(71) Applicant: HOYA Corporation, Tokyo 161-8525 (JP)
(72) Inventor: KOBAYASHI, Shotaro, Tokyo 161-8525 (JP)
(74) Representative: Schaumburg, Thoenes, Thurn, Landskron, Eckert
(86) International application number: PCT/JP2011/070969
(87) International publication number: WO 2012/070298

(57) **Abstract**

A scanning confocal endoscope system is configured by: a point source that scans on a subject with excitation light by periodically moving in a two-dimensional plane; a point source control means that controls the point source so that irradiation density of the excitation light becomes smaller than or equal to predetermined density over a whole scanning area; a confocal pinhole arranged at a position conjugate with a converging point of the excitation light; an image signal detection means that detects an image signal by receiving fluorescence emitted from the subject being excited by the excitation light via the confocal pinhole; and an image generation means that generates a confocal image using the detected image signal.

## Description

### TECHNICAL FIELD

The present invention relates to a scanning confocal endoscope system which detects and images only light, obtained through a pinhole arranged at a position conjugate with a focal point of a confocal optical system, of fluorescence emitted from a subject excited by excitation light.

### BACKGROUND ART

A scanning confocal endoscope system for observing a living tissue in a body cavity is known. The scanning confocal endoscope system scans, with excitation light, on a subject into which medicine is given. The scanning confocal endoscope system detects, with a photodetector, only a component, obtained through a pinhole arranged at a position conjugate with a focal point of a confocal optical system, of light emitted from a subject being scanned. The scanning confocal endoscope system generates an image with a high magnification and a high resolution relative to an image observed through a normal electronic scope or a normal fiber scope, based on a signal generated in response to the intensity of the detected light.

An example of a specific configuration of a scanning confocal endoscope system of this type is described in Japanese Patent Provisional Publication No. JP2004-321792A (hereafter, referred to as "patent document 1"). The scanning confocal endoscope system described in patent document 1 scans on a subject by moving periodically a tip portion of an optical fiber which transmits excitation light and fluorescence.

### SUMMARY OF THE INVENTION

As a scanning manner of excitation light in a scanning confocal endoscope system, a raster scanning manner in which light scans horizontally in one direction within a scanning area is employed. Under such a technical common knowledge in the technical field of the scanning confocal endoscope system, the inventor of the present invention made a specific verification while considering applying another scanning manner to the scanning confocal endoscope system. The scanning manner considered includes a raster scanning manner in which light scans to reciprocate in a horizontal direction within a scanning area, a spiral scanning manner in which light spirally scans from the center toward the periphery within a scanning area and a Lissajous scanning manner in which light scans in a sinusoidal shape within a scanning area.

Through the verification for the new scanning manner, it has become clear that discoloration of fluorescence occurs significantly at a particular portion in an observation area and the portion appears as a dark image in the observation area. If fluorescence is discolored and thereby a shot image becomes blurred, detection of a diseased portion or an accurate judgment on a diseased portion by a doctor may be affected, which is undesirable.

The present invention was made in consideration of the above described circumstances, and the object of the present invention is to provide a scanning confocal endoscope system suitable for suppressing discoloration of fluorescence depending on a scanning manner.

In order to suppress progress of discoloration of fluorescent material contained in medicine for any scanning manner, a scanning confocal endoscope system according to an embodiment of the invention which solves the above described problem comprises: a point source that scans on a subject with excitation light by periodically moving in a two-dimensional plane; a point source control means that controls the point source so that irradiation density of the excitation light becomes smaller than or equal to predetermined density over a whole scanning area; a confocal pinhole arranged at a position conjugate with a converging point of the excitation light; an image signal detection means that detects an image signal by receiving fluorescence emitted from the subject excited by the excitation light via the confocal pinhole; and an image generation means that generates a confocal image using the detected image signal.

The point source control means may control an intensity of the excitation light so that the irradiation density of the excitation light becomes uniform over the whole scanning area. The point source control means may control a duty ratio of the excitation light so that the irradiation density of the excitation light becomes uniform over the whole scanning area.

The image generation means may assign a two dimensional pixel position to each image signal in response to a detection timing of the image signal, and generate the confocal image by spatially arranging point images represented by the image signals in accordance with the assigned pixel positions.

The image generation means may perform assigning the pixel position of each image signal in response to the detection timing and a pixel value calculation using the image signal for each assigned pixel position so that signal values of the pixels defined when a subject having a uniform reflectivity is irradiated with the excitation light become equal to each other.

For example, in the pixel value calculation, at least one of integration, subtraction, multiplication, division, averaging and discarding is performed with respect to the image signals assigned to the pixel positions.

The image signal detection means may control a gain in conjunction with control of an intensity of the excitation light by the point source control means.

For example, scanning of the point source is a spiral scanning where the excitation light scans on the subject in a spiral form from a center to a periphery of the scanning area. During a scanning period of the excitation light, the point source control means increases or decreases linearly or non-linearly an intensity of the excitation light, in conformity with an increase rate of a number of pixel positions in one spiral with respect to a number of times of spirals. In this case, the image generation means may perform the pixel value calculation based on a change rate of the intensity of the excitation light and the increase rate.

During a scanning period of the excitation light, the point source control means may increase linearly the intensity of the excitation light. In the pixel value calculation, values of the image signals assigned to a same pixel position may be integrated.

According to the invention, a scanning confocal endoscope system suitable for suppressing discoloration of fluorescence depending on a scanning manner is provided.

### BRIEF DESCRIPTION OF THE DRAWINGS

[Fig. 1] Fig. 1 is a block diagram illustrating a configuration of a scanning confocal endoscope system according to an embodiment of the invention.
[Fig. 2] Fig. 2 generally illustrates a configuration of a confocal optical unit provided in the scanning confocal endoscope system according to the embodiment of the invention.
[Fig. 3] Fig. 3 illustrates a rotation trajectory of a tip of an optical fiber on an XY approximate plane.
[Fig. 4] Fig. 4 is a diagram relating to the intensity of excitation light emitted from a light source according to the embodiment of the invention.
[Fig. 5] Fig. 5 illustrates diagrams similar to Figs. 4(a) and 4(b) in a first variation.
[Fig. 6] Fig. 6 illustrates diagrams similar to Figs. 4(a) and 4(b) in a second variation.
[Fig. 7] Fig. 7 illustrates diagrams similar to Figs. 4(a) and 4(b) in a third variation.

### EMBODIMENTS FOR CARRYING OUT THE INVENTION

In the following, a scanning confocal endoscope system according to an embodiment of the invention is explained with reference to the accompanying drawings.

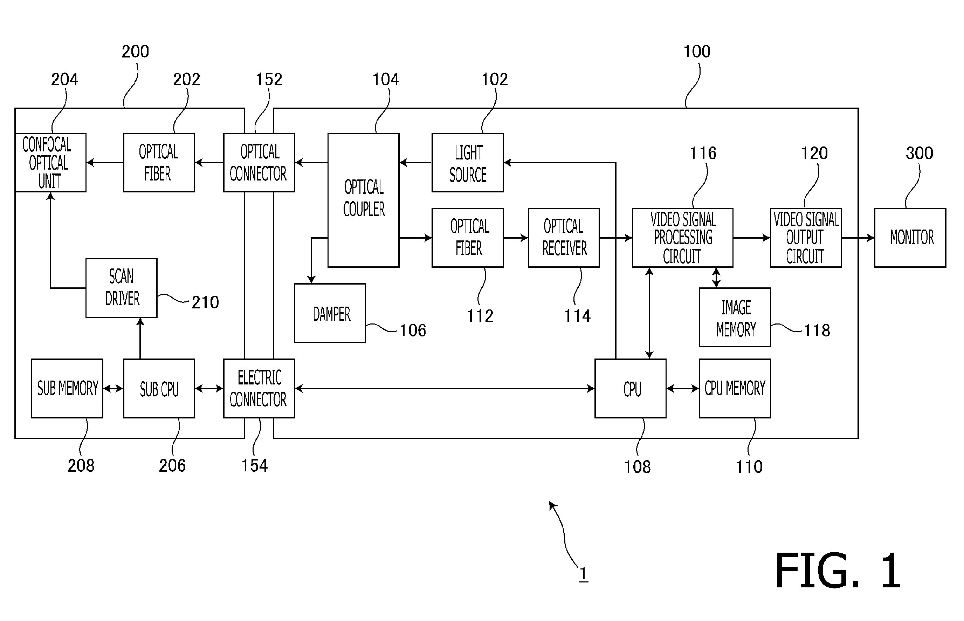

Fig. 1 is a block diagram illustrating a configuration of a scanning confocal endoscope system 1 according to the embodiment of the invention. The scanning confocal endoscope system 1 according to the embodiment of the invention is a system designed by making use of a fundamental principle of a confocal microscope, and is configured suitable for observing a subject at a high magnification and a high resolution. As shown in Fig. 1, the scanning confocal endoscope system 1 includes a system main body 100, a confocal probe 200 and a monitor 300. Confocal observation using the scanning confocal endoscope system 1 is performed in a state where a tip face of the tube-like confocal probe 200 having flexibility is operated to contact a subject.

The system main body 100 includes a light source 102, an optical coupler 104, a damper 106, a CPU 108, a CPU memory 110, an optical fiber 112, an optical receiver 114, a video signal processing circuit 116, an image memory 118 and a video signal output circuit 120. The confocal probe 200 includes an optical fiber 202, a confocal optical unit 204, a sub CPU 206, a sub memory 208 and a scan driver 210.

The light source 102 emits excitation light which excites medical agents administered in a body cavity of a patient in accordance with driving control by the CPU 108. The excitation light enters the optical coupler 104. To one of ports of the optical coupler 104, an optical connector 152 is coupled. To a non-use port of the optical coupler 104, the damper 106 which terminates, without reflection, the excitation light emitted from the light source 102 is coupled. The excitation light which has entered the former port passes through the optical connector 152, and enters an optical system arranged in the confocal probe 200.

A proximal end of the optical fiber 202 is optically coupled to the optical coupler 104 through the optical connector 152. A tip of the optical fiber 202 is accommodated in the confocal optical unit 204 which is installed in a tip portion of the confocal probe 200. The excitation light which has exited from the optical coupler 104 enters the proximal end of the optical fiber 202 after passing through the optical connector 152, passes through the optical fiber 202, and thereafter is emitted from the tip of the optical fiber 202

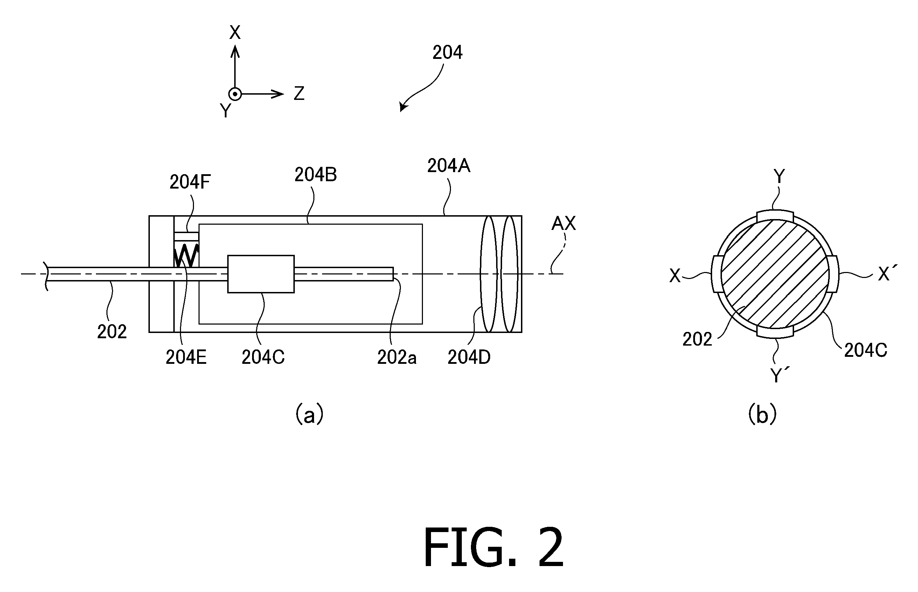

Fig. 2(a) generally illustrates a configuration of the confocal optical unit 204. In the following, for convenience of explanation for the confocal optical unit 204, a direction of the longer side of the confocal optical unit 204 is defined as Z-direction, and the two directions which are perpendicular to the Z-direction and are perpendicular with respect to each other are defined as X-direction and Y-direction. As shown in Fig. 2(a), the confocal optical unit 204 has a metal outer tube 204A which accommodates various components. The outer tube 204A holds, to be slidable in a coaxial direction, an inner tube 204B having an outer wall shape corresponding to an inner wall shape of the outer tube 204A. The tip (a reference symbol 202a is assigned hereafter) of the optical fiber 202 is accommodated and supported in the inner tube 204B through openings formed in proximal end faces of the outer tube 204A and the inner tube 204B, and functions as a secondary point source of the scanning confocal endoscope system 1. The position of the tip 202a being the point source changes periodically under control by the CPU 108.

The sub memory 208 stores probe information, such as identification information and various properties of the confocal probe 200. The sub CPU 206 reads out the probe information from the sub memory 208 at a time of start-up, and transmits the probe information to the CPU 108 via an electric connector 154 which electrically connects the system main body 100 with the confocal probe 200. The CPU 108 stores the transmitted probe information in the CPU memory 110. The CPU 108 generates signals necessary for controlling the confocal probe 200 by reading out the stored probe information when necessary, and transmits the signals to the sub CPU 206. The sub CPU 206 designates setting values required for the scan driver 210 in accordance with the control signals from the CPU 108.

The scan driver 210 generates a drive signal corresponding to the designated setting value, and drives and controls a biaxial actuator 204C adhered and fixed to the outer surface of the optical fiber 202 close to the tip 202a. Fig. 2(b) generally illustrates a configuration of the biaxial actuator 204C. As shown in Fig. 2(b), the biaxial actuator 204C is a piezoelectric actuator in which a pair of X-axis electrode (X and X' in the figure) and Y-axis electrode (Y and Y' in the figure) connected to the scan driver 210 are formed on a piezoelectric body.

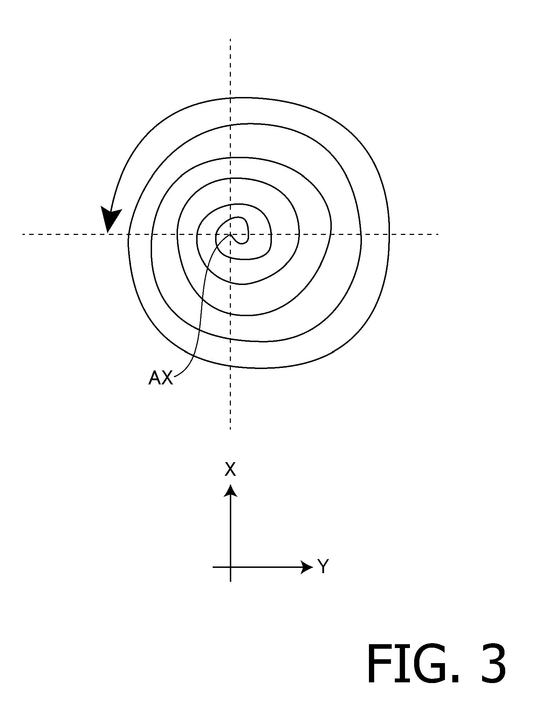

The scan driver 210 applies an alternating voltage X between the electrodes for the X-axis of the biaxial actuator 204C so that the piezoelectric body is resonated in the X-direction, and applies an alternating voltage Y which has the same frequency as that of the alternating voltage X and has a phase orthogonal to the phase of the alternating voltage X, between the electrodes for the Y-axis so that the piezoelectric body is resonated in the Y-axis direction. The alternating voltage X and the alternating voltage Y are defined as voltages which linearly increase in amplitude in proportion to time and reach average root-mean-square values (X) and (Y) by taking times (X) and (Y), respectively. The tip 202a of the optical fiber 202 rotates to draw a spiral pattern having the center at the center axis AX on a plane (hereafter, referred to as a "XY approximate plane") which approximates the X-Y plane, due to combining of kinetic energies in the X- direction and Y-direction by the biaxial actuator 204C. A rotation trajectory of the tip 202a becomes larger in proportion to the applied voltage, and becomes a circle having the maximum diameter when the alternating voltages having the average root-mean squares (X) and (Y) are applied. Fig. 3 illustrates the rotation trajectory of the tip 202a on the XY approximate plane.

The excitation light is continuous light, and is emitted from the tip 202a of the optical fiber 202 during a time period from a time immediately after start of application of the alternating voltage to the biaxial actuator 204C to a time of stop of the application of the alternating voltage. In the following, for convenience of explanations, this time period is referred to as a "sampling period". When the application of the alternating voltage to the biaxial actuator 204C is stopped after the sampling period has elapsed, vibration of the optical fiber 202 attenuates. The circular motion of the tip 202a on the XY approximate plane converges in accordance with convergence of the vibration of the optical fiber 202, and stops on the center axis AX after passing of a predetermined time. In the following, for convenience of explanations, a time period from a time of the end of the sampling period to a time of stop of the tip 202a on the center axis AX (more specifically, a time period slightly longer than a mathematically defined time period elapsing until stop of the tip 202a, in order to ensure stop of the tip 202a on the center axis AX) is referred to as a "braking period". A time period corresponding to one frame is formed of one sampling period and one braking period. In order to shorten the braking period, a reverse phase voltage may be applied to the biaxial actuator 204C at an initial stage of the braking period so as to positively apply a braking torque.

On the front side of the tip 202a of the optical fiber 202, an objective optical system 204D is installed. The objective optical system 204D is formed of a plurality of optical lenses, and is held in the outer tube 204A via a lens frame not shown. In the outer tube 204A, the lens frame is supported and fixed with respect to the inner tube 204B. Therefore, a lens group held on the lens frame slides along the Z-direction together with the inner tube 204B in the outer tube 204A.

Between a proximal end face of the inner tube 204B and the inner wall of the outer tube 204A, a helical compression spring 204E and a shape memory alloy 204F are attached. The helical compression spring 204E is initially compressed and sandwiched in the Z-direction from a natural length thereof. The shape memory alloy 204F has a rod-like shape elongated in the Z-direction, deforms when an external force is applied thereto under a room temperature condition, and is restored to a predetermined shape by the shape memory effect when heated to be higher than or equal to a predetermined temperature. The shape memory alloy 204F is designed such that the restoring force by the shape memory effect is larger than the restoring force of the helical compression coil 204E. The scan driver 210 generates a driving signal corresponding to the setting value designated by the sub CPU 206, and controls the expanding and contracting amount of the shape memory alloy 204F by electrifying and heating the shape memory alloy 204F. The shape memory alloy 204F causes the inner tube 204B to move forward or backward in the Z-direction in accordance with the expanding and contracting amount.

The excitation light emitted from the tip 202a of the optical fiber 202 forms a spot on a surface or a surface layer of the subject through the objective optical system 204D. A spot formation position shifts in the Z-direction depending on movement of the tip 202a being the point source. That is, the confocal optical unit 204 performs the three dimensional scanning on the subject by combining the periodic circular motion of the tip 202a on the XY approximate plane by the biaxial actuator 204C and the movement in the Z-axis direction.

Since the tip 202a of the optical fiber 202 is arranged at the front focal point of the objective optical system 204D, the tip 202a functions as a confocal pinhole. Of the scattered component (fluorescence) of the subject excited by the excitation light, only fluorescence from the convergence point which is optically conjugate with the tip 202a is incident on the tip 202a. The fluorescence passes through the optical fiber 202, and then enters the optical coupler 104 through the optical connector 152. The optical coupler 104 separates the entered fluorescence from the excitation light emitted from the light source 102, and guides the fluorescence to the optical fiber 112. The fluorescence is transmitted through the optical fiber 112, and then is detected by the optical receiver 114. In order to detect feeble light with a low level of noise, the optical receiver 114 may be configured as a high-sensitivity optical detector, such as a photomultiplier.

The detection signal is inputted to the video signal processing circuit 116. The video signal processing circuit 116 operates under control of the CPU 108, and generates a digital detection signal by performing sampling-and-holding and AD conversion for the detection signal at a constant rate. When the position (trajectory) of the tip 202a of the optical fiber 202 during the sampling period is determined, the spot formation position in the observation area (the scanning area) corresponding to the determined position and the signal acquisition timing for obtaining the digital detection signal by detecting the returning light from the spot formation position are definitely defined. In this embodiment, the spot formation position is estimated in advance from the signal acquisition timing with reference to experimental results using a calibration tool, and a position on an image corresponding to the estimated position is determined. In the CPU memory 110, a remapping table in which the determined signal acquisition timings are associated with pixel positions (pixel addresses) is stored.

The video signal processing circuit 116 refers to the remapping table, and assigns a point image represented by the digital detection signal to a pixel address in response to the signal acquisition timing. In the following, the above described assigning work is referred to as remapping, for convenience of explanation. In accordance with results of the remapping, the video signal processing circuit 116 performs buffering by storing the signal of the image formed by the spatial arrangement of point images into the image memory 118 on a frame-by-frame manner. The buffered signal is swept out at a predetermined timing from the image memory 118 to the video signal output circuit 120, and is displayed on the monitor 300 after being converted into a video signal complying with a predetermined standard, such as NTSC (National Television System Committee) or PAL (Phase Alternating Line). On a display screen of the monitor 300, a three-dimensional confocal image with a high magnification and a high resolution is displayed.

Incidentally, the subject is scanned in a spiral form (the spiral scanning) from the center toward the periphery of the scanning area in regard to the XY directions. The scanning trajectory with respect to the subject is a spiral trajectory as in the case of Fig. 3. Since the optical fiber 202 produces a resonance motion, a cycle (a time period for one rotation scan) of each spiral is constant. Since the irradiation density (the irradiation energy per a unit area) of the excitation light becomes higher at a point closer to the center, decomposition of a fluorescent body progresses faster at a point closer to the center and discoloration occurs. As a result, a problem arises that an image becomes dark at a central portion of an observation area in which an observation target lies. In order to suppress discoloration of fluorescence, a measure of decreasing the intensity of the excitation light can be considered, for example. However, if the intensity of the excitation light is decreased, a noise stands out particularly at the peripheral portion in the observation area due to shortage of the detection light amount. In either case, detection of a diseased portion or an accurate judgment on a diseased portion by a doctor may be affected, which is undesirable. For this reason, the scanning confocal endoscope system 1 according to the embodiment is configured to suitably suppress discoloration of fluorescence by appropriately controlling the intensity (or the light amount) of the excitation light.

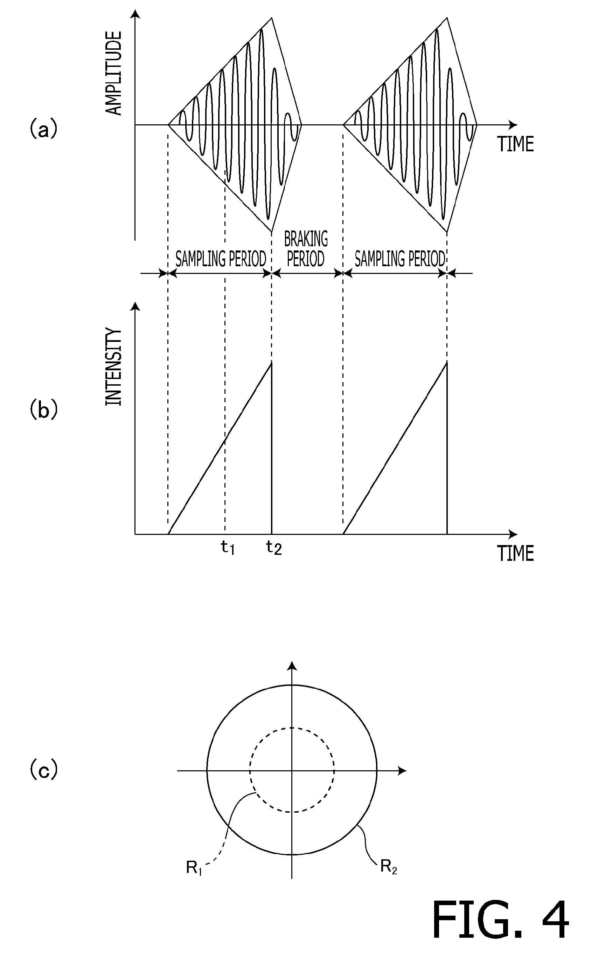

Fig. 4(a) illustrates the motion of the tip 202a of the optical fiber 202. Fig. 4(b) illustrates the intensity of the excitation light emitted from the light source 102. The horizontal axis of each of Figs. 4(a) and 4(b) is the time axis. The vertical axis of Fig. 4(a) represents the shift amount in the X-direction (or Y-direction) of the tip 202a with reference to the center axis AX. The vertical axis of Fig. 4(b) represents the intensity of the excitation light. As shown in Fig. 4(b), immediately after moving to the sampling period, the intensity of the excitation light is zero. The CPU 108 linearly increases, from zero, the intensity of the excitation light from the start of the sampling period to the end of the sampling period. The excitation light is set such that the irradiation density is smaller than or equal to a predetermined density over the whole scanning area. Since the irradiation density of the excitation light decreases in the central portion of the scanning area, the discoloration of fluorescence can be suppressed. Since decrease of the irradiation density of the excitation light is suppressed in the peripheral portion of the scanning area, deterioration of the SN ratio due to shortage of the detection light amount is small in the peripheral portion of the scanning area. On the other hand, there is a concern that the SN ratio decreases in the central portion of the observation area due to decrease of the irradiation density of the excitation light in the central portion of the scanning area.

Fig. 4(c) schematically illustrates the spiral trajectory of the excitation light at the time t₁ and the time t₂ in Figs. 4(a) and 4(b). In Fig. 4(c), a spiral at the time t₁ is assigned a reference symbol "R₁", and a spiral at the time t₂ is assigned a reference symbol "R₂". In this embodiment, the number of samples of digital detection signals obtained during one spiral is 2000, and the number of pixel addresses respectively assigned to the digital detection signals obtained in the spirals R₁ and R₂ are 500 and 2000. The number of pixel addresses corresponding to the spot formation positions on a spiral becomes larger as the diameter of the spiral increases (i.e., as a trajectory becomes long) because the pixels are arranged to have constant intervals in a matrix. At the time t₁, four (=2000/500) digital detection signals are assigned to one pixel address. Since the value of one pixel is an integrated value of four digital detection signals, decrease of SN ratio due to decrease of the irradiation density of the excitation light can be suppressed. Since the intensity of the excitation light is low and the value of each digital detection signal is small, the pixel value (the integrated value) does not saturate. At the time t₂, one digital detection signal is assigned to one pixel address. Since the intensity of the excitation light is high and the value of the digital detection signal is high, the SN ratio is high.

The number of pixel addresses assigned to one spiral does not necessarily increase linearly with respect to the number of times of spirals. Therefore, the pixel value may be calculated by appropriately combining subtraction, multiplication, division and averaging, while considering the relationship between the increase rate of the intensity of the excitation light and the increase rate of the number of pixel addresses with respect to the number of times of spirals, without limiting to the integration. The calculation of the pixel value may be set such that the sensitivity (a signal value of each pixel defined when the excitation light is irradiated to a subject having a uniform reflectivity) becomes the same for all of the pixels.

During the sampling period, the intensity of the excitation light may be increased or decreased linearly or nonlinearly in conformity with the increase rate of the number of pixel addresses with respect to the number of times of spirals. In this case, the intensity of the excitation light may be set such that the irradiation density becomes uniform over the whole scanning area, for example.

### (First Variation)

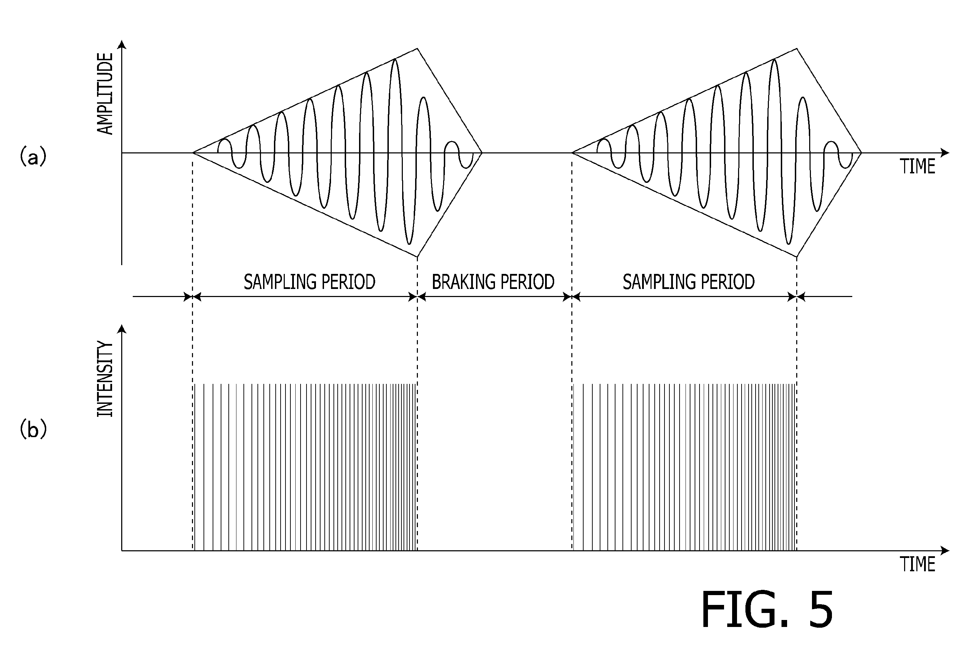

Figs. 5(a) and 5(b) are diagrams similar to Figs. 4(a) and 4(b), in a first variation of the scanning confocal endoscope system 1 according to the embodiment. Configurations of variations explained hereafter have the same block configuration as that of the above described embodiment. Therefore, detailed explanations of hardware and software configurations of variations are simplified or omitted.

As shown in Fig. 5(b), in the first variation, the duty ratio of the excitation light continuously increases from the start to the end of the sampling period. The duty ratio may be set such that the pixel position and the spot formation position of the excitation light (pulse light) are determined in one-to-one relationship (i.e., one pulse per one pixel, and the irradiation density is uniform over the whole scanning area). In the first variation, the intensity itself of the excitation light is constant during the sampling period. However, in the central portion of the scanning area, the energy absorbed by the fluorescence body decreases temporally. Since progress of the decomposition of the fluorescence body delays, the discoloration of the fluorescence can be suppressed. Since decrease of the irradiation density of the excitation light can be suppressed in the peripheral portion of the scanning area, deterioration of the SN ratio due to shortage of the detection light amount is small in the peripheral portion of the observation area.

### (Second Variation)

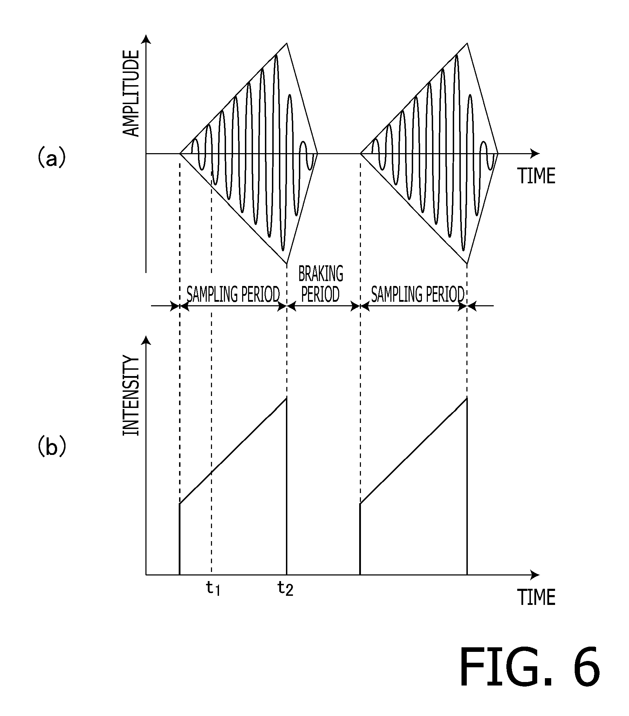

Figs. 6(a) and 6(b) are diagrams similar to Figs. 4(a) and 4(b) in a second variation of the scanning confocal endoscope system 1 according to the embodiment. In the second variation, the light source 102 is a laser diode.

As in the case of the embodiment, in the second variation, the intensity of the excitation light increases from the start to the end of the sampling period. In order to stabilize output of the laser diode, a certain degree of power is required. Therefore, in contrast to the embodiment, the intensity of the excitation light immediately after moving to the sampling period is not zero. Since, in the second variation, the irradiation density of the excitation light also decreases in the central portion of the scanning area, the discoloration of fluorescence can be suppressed. Since decrease of the irradiation density of the excitation light can be suppressed in the peripheral portion of the scanning area, deterioration of the SN ratio due to shortage of the detection light amount is small in the peripheral portion of the observation area.

In the second variation, the intensity of the excitation light at the time t₁ is high in comparison with the embodiment. For this reason, there is a possibility that the pixel value is saturated when four digital detection signals are integrated. Therefore, in the second variation, the pixel value is calculated by using integration and division at the same time. Alternatively, the pixel value may be obtained by discarding at least one digital detection signal and by integrating the remaining digital detection signals.

### (Third Variation)

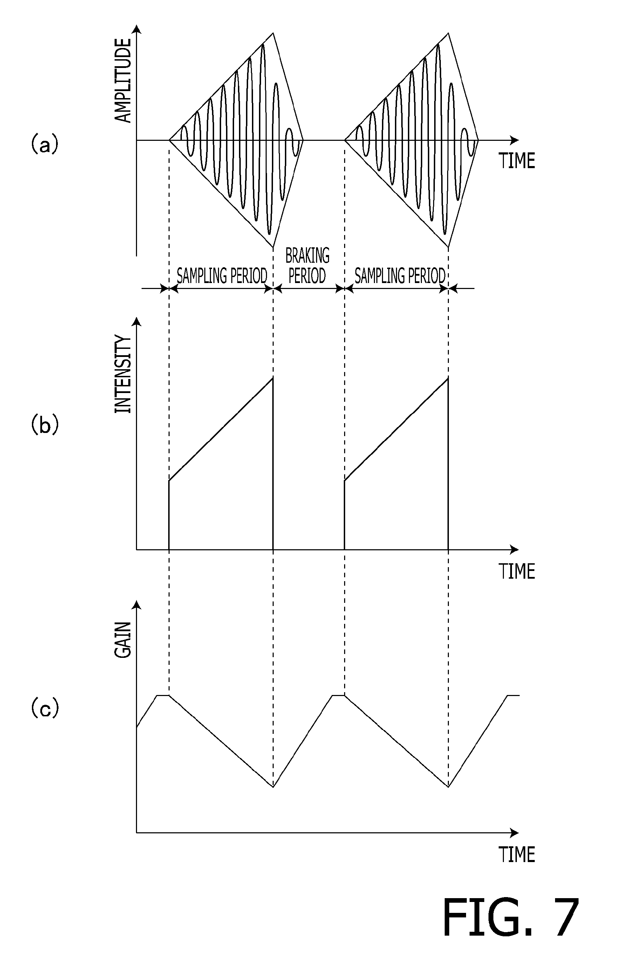

Figs. 7(a) and 7(b) are diagrams similar to Figs. 4(a) and 4(b) in a third variation of the scanning confocal endoscope system 1 according to the embodiment. Fig. 7(c) illustrates a gain of the optical receiver 114. In Fig. 7(c), the horizontal axis is a time axis, and the vertical axis represents the gain.

Control of the light source 102 is the same as that of the second variation. In the third variation, the gain of the optical receiver 114 is set to a high value immediately after moving to the sampling period. Therefore, even if the speed of the spiral scanning is increased, for example, due to increase of the number of pixels or the frame rate, there is no concern about the shortage of the detection light amount in the central portion of the scanning area where the irradiation density of the excitation light decreases. The gain of the optical receiver 114 is controlled to decrease as the intensity of the excitation light increases. Therefore, saturation of the pixel value in the peripheral portion of the observation area can be effectively avoided.

The foregoing is the explanations of the embodiment of the invention. The invention is not limited to the above described configuration, but can be varied in various ways within the scope of the technical concept of the invention. For example, control of the light source 102 may be performed by combining change of the duty ratio of the excitation light and change of the intensity of the excitation light.

The scamming manner which can be applied to the invention is not limited to the spiral scanning manner. For example, the invention may be applied to a scanning confocal endoscope system which employs a raster scanning manner in which light scans to reciprocate in a horizontal direction within a scanning area or a Lissajous scanning manner in which light scans in a sinusoidal shape within a scanning area. That is, in other various scanning manners, the irradiation density may become high at a particular portion in the observation area and discoloration of fluorescence may progress. Such a problem can also be effectively solved by applying the invention thereto and thereby performing the above described light source control and the various calculation processes.

## Claims

1. A scanning confocal endoscope system, comprising:
a point source that scans on a subject with excitation light by periodically moving in a two-dimensional plane;
a point source control means that controls the point source so that irradiation density of the excitation light becomes smaller than or equal to predetermined density over a whole scanning area;
a confocal pinhole arranged at a position conjugate with a converging point of the excitation light;
an image signal detection means that detects an image signal by receiving fluorescence emitted from the subject excited by the excitation light via the confocal pinhole; and
an image generation means that generates a confocal image using the detected image signal.

2. The scanning confocal endoscope system according to claim 1, wherein the point source control means controls an intensity of the excitation light so that the irradiation density of the excitation light becomes uniform over the whole scanning area.

3. The scanning confocal endoscope system according to claim 1 or 2, wherein the point source control means controls a duty ratio of the excitation light so that the irradiation density of the excitation light becomes uniform over the whole scanning area.

4. The scanning confocal endoscope system according to any of claims 1 to 3, wherein the image generation means assigns a two dimensional pixel position to each image signal in response to a detection timing of the image signal, and generates the confocal image by spatially arranging point images represented by the image signals in accordance with the assigned pixel positions.

5. The scanning confocal endoscope system according to claim 4, wherein the image generation means performs assigning the pixel position of each image signal in response to the detection timing and a pixel value calculation using the image signal for each assigned pixel position so that signal values of the pixels defined when a subject having a uniform reflectivity is irradiated with the excitation light become equal to each other.

6. The scanning confocal endoscope system according to claim 5, wherein, in the pixel value calculation, at least one of integration, subtraction, multiplication, division, averaging and discarding is performed with respect to the image signals assigned to the pixel positions.

7. The scanning confocal endoscope system according to any of claims 1 to 6, wherein the image signal detection means controls a gain in conjunction with control of an intensity of the excitation light by the point source control means.

8. The scanning confocal endoscope system according to any of claims 1 to 7,
wherein;
scanning of the point source is a spiral scanning where the excitation light scans on the subject in a spiral form from a center to a periphery of the scanning area; and
during a scanning period of the excitation light, the point source control means increases or decreases linearly or non-linearly an intensity of the excitation light, in conformity with an increase rate of a number of pixel positions in one spiral with respect a number of times of spirals.

9. The scanning confocal endoscope system according to claim 8 which refers to claim 5, wherein the image generation means performs the pixel value calculation based on a change rate of the intensity of the excitation light and the increase rate.

10. The scanning confocal endoscope system according to any of claims 1 to 7,
wherein:
scanning of the point source is a spiral scanning where the excitation light scans on the subject in a spiral form from a center to a periphery of the scanning area; and
during a scanning period of the excitation light, the point source control means increases linearly the intensity of the excitation light.

11. The scanning confocal endoscope system according to claim 10 which refers to claim 5, wherein, in the pixel value calculation, values of the image signals assigned to a same pixel position are integrated.
